# EUROPÄISCHE PATENTSCHRIFT

(11) **EP 3 772 362 B1**
(45) Veröffentlichungstag und Bekanntmachung des Hinweises auf die Patenterteilung: **21.12.2022**
(21) Anmeldenummer: 20189619.8
(22) Anmeldetag: 05.08.2020
(51) Int. Cl.: A61N 2/00, A61N 2/02

(54) **VORRICHTUNG ZUR MAGNETFELDSTIMULATION VON KÖRPERGEWEBE EINES BENUTZERS ZUR VERWENDUNG IN DER DIGITALEN TELEMEDIZIN, INSBESONDERE NACH DER E-PULS-METHODE ZUR BECKENBODENOPTIMIERUNG**
DEVICE FOR MAGNETIC FIELD STIMULATION OF BODY TISSUE OF A USER FOR USE IN DIGITAL TELEMEDICINE, IN PARTICULAR ACCORDING TO THE E-PULS METHOD FOR PELVIC FLOOR OPTIMISATION
DISPOSITIF DE STIMULATION PAR CHAMP MAGNÉTIQUE DU TISSU CORPOREL D'UN UTILISATEUR DESTINÉS À ÊTRE UTILISÉS EN TÉLÉMÉDECINE NUMÉRIQUE, EN PARTICULIER SELON LE PROCÉDÉ D'E-PULSE PERMETTANT L'OPTIMISATION DU PLANCHER PELVIEN

(30) Priorität: 05.08.2019 DE 102019121072
(43) Veröffentlichungstag der Anmeldung: 10.02.2021
(73) Patentinhaber: Prof. Dr. Fischer AG, 9497 Eschen (LI)
(72) Erfinder: FISCHER, Gerhard, 9435 Heerbrugg (CH)
(74) Vertreter: Riebling, Peter

(56) Entgegenhaltungen:
- EP-A2- 2 676 700
- CN-A- 109 381 790
- DE-A1-102017 122 942
- KR-A- 20160 044 184

## Beschreibung

Die Erfindung betrifft eine Vorrichtung zur Magnetfeldstimulation von Körpergewebe eines Benutzers, insbesondere nach der E-Puls-Methode zur Beckenbodenoptimierung. Die Vorrichtung zur Magnetfeldstimulation ist insbesondere geeignet für die Verwendung in der digitalen Telemedizin.

Die Vorrichtung dient der Stimulation von Motoneuronen sowie des Reflexbogens durch Afferenzen des peripheren Nervensystems, um Skelettmuskeln des Beckens zur Kontraktion zu bringen und durch Stimulation des N. pudendus die Reflexantwort des M. detrusor vesicae zu hemmen. Die Vorrichtung ist dabei insbesondere auch zur Behandlung einer Beckenbodenschwäche und einer damit zusammenhängenden Inkontinenzsymptomatik, Reizzuständen der Blase (OAB-Syndrom / Dranginkontinenz) und zur Behandlung erektiler Dysfunktionen des blutabflussbedingten Formenkreises angelegt. Sie ist darüber hinaus für eine Vielzahl prophylaktischer und therapeutischer Anwendungen im physiotherapeutischen, orthopädischen sowie dermatologischen Bereich ausgelegt.

Mit dem E-Pulssystem wurden klinische Studien sowie eine Pflegestudie (Schrank S, Adlbrecht L, Mayer H. Repetitive periphere Muskelstimulation vs. Beckenbodentraining. Z Gerontol Geriat. 2018. 51: 675-681) durchgeführt. Um systemimmanente Fehler auszuschließen (Bias), wie sie selbst bei randomisierten und kontrollierten Doppelblindstudien nicht auszuschließen sind, wurde eine repräsentative Studie mit insgesamt 120 Teilnehmerinnen (Belastungsinkontinenz) unter Maßgabe der Oxford Skala (Jadad) durchgeführt (Jadad AR, Moore RA, Carroll D et al. Assessing the quality of reports of randomized clinical trials: Is blinding necessary?. Controlled Clinical Trials. 1996; 17 (1): 1-12) und erzielte hier den höchstmöglich erreichbaren Jadad-Score 5 (Lim R, Liong ML, Leong WS et al. Pulsed magnetic stimulation for stress incontinence: 1-year followup results. J Urology. 2017 May; 197: 1-7. Der validierte Jadad-Score bezieht sich dabei auf die Methodik einer klinischen Studie bzw. auf deren Randomisierung, Verblindung und Beschreibung der Patientenverläufe. Dem wurde bereits in der Studienplanung Rechnung getragen, indem das Studienprotokoll in einer eigenen Publikation zur Veröffentlichung kam (Lim R, Liong ML, Leong WS et al. Trials 2015 Jun; 16: 279 doi. 10.1186/s13063-015-0803-1). Ergebnis: Nach nur 16 Behandlungen (2 x wöchentlich) kam es bei 75 % der Probandinnen zu einer deutlichen Besserung ("Responder") und sank das durchschnittliche Pad-Gewicht (1-Std. Pad-Test von 11,00 g auf 3,02 g (Placebogruppe 11,57 g auf 9,77 g). 41,7 % waren danach sogar komplett trocken. Die Behandlung zeichnete sich auch dadurch aus, dass auch noch 14 Monate nach Studienbeginn, immer noch 68,4 % der Frauen im zuvor erreichten Responderstatus befanden.

Aus der DE 298 08 990 U1 ist ein Sitz- und Liegemöbel für die Magnetfeldtherapie bekannt, welches eine oder mehrere Applikatorvorrichtungen zur Erzeugung von Magnetfeldern aufweist. Die Applikatorvorrichtung kann dabei in die Polsterung des jeweiligen Sitz- oder Liegemöbels eingebettet sein. Ferner kann die Applikatorvorrichtung zur Erzeugung von Magnetfeldern mit Spulen ausgestattet sein, die eine Ganzkörper- oder eine Teilkörpertherapie mit in Reihen oder kreisförmig angeordneten Vollkern-Metallspulen ermöglicht.

Die DE 10 2017 122 942 A1 betriff eine Entspannungsliege zur entspannenden und/oder therapeutischen Behandlung eines Benutzers, mit einer Liegefläche, welche eine Sitzfläche, eine Rückenlehne und eine Beinstütze aufweist, wobei Mittel zur Erzeugung eines magnetischen Schwingungsfeldes im Bereich der Liegefläche und Mittel zur Erzeugung einer Wiegebewegung und/oder Schaukelbewegung der Liegefläche vorhanden sind.

Aus EP2676700 A2 ist ein Therapiesitz für das maschinengestützte invasive Beckenbodentraining der Beckenboden-Muskulatur mit der transpelvinen Magnetstimulation (TPM) bekannt.

Es ist die Aufgabe der vorliegenden Offenbarung eine Vorrichtung und ein Verfahren zur Magnetfeldstimulation anzugeben, die eine komfortable und benutzerindividuelle Magnetfeldbehandlung erlauben.

Die Vorrichtung zur Magnetfeldstimulation von Körpergewebe eines Benutzers umfasst eine einstellbare Sitzgelegenheit für den Benutzer, mindestens einen Magnetfeldapplikator zur Erzeugung eines pulsierendes Magnetfeldes im Bereich der Sitzgelegenheit, Einstellmittel zur Einstellung eines oder mehrerer Parameter des pulsierenden Magnetfeldes umfassend Pulsdauer, Pulsfrequenz und Pulsamplitude, und/oder zur Einstellung der Sitzgelegenheit, und Anzeigemittel zur Anzeige von Informationen für Information.

Erfindungsgemäß sind die Einstellmittel derart ausgebildet, um einen oder mehrere der Parameter des Magnetfeldes und/oder die Einstellung der Sitzgelegenheit in Abhängigkeit von zuvor aufgenommenen individuellen Informationen über den Benutzer manuell und/oder automatisch einzustellen.

Der Magnetfeldapplikator umfasst einen programmierbaren Impulsgenerator, dessen Pulsdauer, Pulsfrequenz und Pulsamplitude einstellbar sind. Der Impulsgenerator erzeugt niederfrequente Impulse, vorzugsweise im Bereich von 1-100 Hz, und ist mit einer oder mehreren Magnetspulen 15 verbunden, die in der Sitzfläche und/oder der Rückenlehne und/oder dem Fußteil der Sitzgelegenheit ein pulsierendes Magnetfeld erzeugen.

In einer bevorzugten Ausgestaltung der Erfindung erfolgen die Einstellung der Sitzgelegenheit und/oder die Einstellung des pulsierenden Magnetfeldes automatisch anhand der über den Benutzer verfügbaren Informationen.

Zur Feststellung, welcher Benutzer die Vorrichtung benutzen möchte ist es in einer bevorzugten Ausgestaltung der Erfindung vorgesehen, dass eine Vorrichtung zur automatischen Erkennung des Benutzers vorhanden ist. Die Erkennung des Benutzers kann auf optischem und/oder akustischem Weg erfolgen, beispielsweise durch Gesichtserkennung mittels Kamera, Fingerabdruckerkennung oder Stimmerkennung oder einer Kombination dieser Verfahren. Eine andere Möglichkeit besteht in der Erkennung einer Identitätsinformation des Benutzers in Form einer Chipkarte, eines RFID-Chips oder ähnlichem.

Ferner ist es erfindungsgemäß vorgesehen, dass die Einstellmittel zur Eingabe und/oder zum Auslesen von individuellen Informationen über den Benutzer vorhanden sind, wobei die Informationen insbesondere Angaben über die körperlichen Merkmale und/oder den Gesundheitszustand des Benutzers umfassen. Diese Einstellmittel können wahlweise eine Tastatur und/oder einen Touchscreen und/oder eine Vorrichtung zur Spracheingabe umfassen.

In einer anderen bevorzugten Ausgestaltung der Erfindung werden die individuellen Informationen des aktuellen Benutzers mit Informationen von anderen Benutzern verglichen, und es werden die Parameter des Magnetfeldes für den aktuellen Benutzer unter Berücksichtigung der bei anderen Benutzern eingestellten Parameter eingestellt. Mit andern Worten wird eine bei anderen Benutzern erfolgreich durchgeführte Magnetfeldbehandlung auch bei dem aktuellen Benutzer mit ähnlicher Diagnose oder Beschwerden durchgeführt.

Dem Benutzer können auf dem Anzeigemittel Informationen über die Wirkungsweise von unterschiedlichen Einstellungen des pulsierenden Magnetfeldes auf das Körpergewebe dargestellt werden. Insbesondere können sich die Informationen ebenso auf einzelne, mit der Vorrichtung behandelbare Indikationen beziehen, und es können die meist komplexen urologischen und gynäkologischen Veränderungen und Defizite des Beckenbodens, der Blase und des Blasenverschlusssystems anschaulich und prägnant erklärt werden

In einer besonderen Ausgestaltung der Erfindung ist eine Vorrichtung zur Erzeugung von Beleuchtungs- und Lichteffekten vorgesehen, um das Wohlbefinden des Benutzers während der Behandlung zu verbessern und den Behandlungserfolg zu steigern.

Ebenfalls kann ein Infotainmentsystem zum Abspielen von Musik, Ton- und Klangeffekten vorgesehen sein, das der Information und dem Wohlbefinden des Benutzers dient.

Es kann ferner ein Mikrophon zur Aufnahme von Sprachinformationen zur Sprachsteuerung und/oder zur Erkennung des Benutzers (Stimmerkennung) vorgesehen sein.

Die Bedienvorrichtung kann eine Tastatur und/oder eine berührungsempfindliche Anzeigevorrichtung (Touchscreen) aufweisen.

Die vom Benutzer aufgenommenen persönlichen Informationen werden vorzugsweise in der Vorrichtung selbst und/oder auch in einer Zentrale gespeichert und ausgewertet. Die Zentrale kann ein telemedizinisches Zentrum sein, welches die Benutzerdaten auswertet und auf Wunsch unterstützende Informationen für die Behandlung des Benutzers liefert. Die Vorrichtung zur Magnetfeldbehandlung ist über ein Kommunikationssystem mit der Zentrale verbunden.

Ferner sind in einer bevorzugten Weiterbildung der Erfindung im Bereich der Sitzgelegenheit Haltegriffe für den Benutzer vorhanden. Damit kann der Benutzer während der Behandlung aktiv seine Muskulatur anspannen und den Behandlungserfolg steigern.

Die Vorrichtung kann ferner derart ausgebildet sein, dass die in der Sitzfläche der Vorrichtung vorhandene Magnetspule in Bezug auf die Sitzfläche beweglich angeordnet ist. Die Bewegung der Magnetspule in Bezug auf die Sitzfläche erfolgt automatisch während der Magnetfeldstimulationsbehandlung durch einen Antrieb, wobei die Bewegung der Magnetspule automatisch zwischen zwei oder mehreren Endpositionen erfolgt, also z.B. in Sitzrichtung des Benutzers vor und zurück oder nach links und rechts, oder einem vorgegeben Bewegungspfad folgend gesteuert ist, z.B. einer Kreisbahn folgt.

Dies erfolgt vorrangig, um einer muskulären Überlastung entgegen zu steuern und ein Therapieoptimum zu erreichen,. So werden z.B. - in der Mitte des Beckenbodens beginnend - zuerst die hinteren und danach die vorderen Beckenbodenanteile in einer zeitlichen Taktung abgefahren und zudem noch durch mehrmalige Pausen von z.B. 30 Sekunden Dauer unterbrochen. Unabhängig einer generellen, intensitätsabhängigen Globalkontraktion, werden damit auch einzelne, definierte Muskelanteile besonders stark stimuliert, ohne dass eine Überlastung oder Ernährungsstörung des Muskels zu erwarten ist. Gleichzeitig erlaubt es die Chipkartensteuerung, den für den Erektionserhalt wichtigen M. ischiocavernosus oder die für die weibliche Orgasmusförderung notwendigen M. pubococygeus und M. ileococcygeus gezielt anzusprechen. Auch gelingt es damit, eine direkte Stimulation des Wachstums- und Reparaturfaktors BDNF (Brain-Derived Neutrophic Factor) des im Geburtskanal (Levtortor) verlaufenden Nervus pudendus vorzunehmen und so für eine Frührehabilitation (Post-Partum-Training) dieses bei der Geburt geschädigten Nervs zu sorgen. Damit lässt sich nicht nur die für ein optimales Muskelzellwachstum erforderliche Therapiezeit exakt bestimmen und verkürzen, sondern wird auch der Therapeut oder das betreuende Assistenzpersonal eines rPMS-Trainings komplett entlastet. Denn sämtliche Indikationen eines rPMS laufen damit selbsttätig ab und bedürfen keiner weiteren gerätetechnischen Intervention

Begründung: Erfahrungen aus einem aktiven Beckenbodentraining legen nahe, dass die Beckenbodenmuskulatur bei einer isolierten, isometrischen Anspannung nur einen geringen Teil ihrer möglichen Kraft produzieren kann. Im Gegensatz dazu scheint durch eine Elektrostimulation - und damit auch bei einer rPMS - die Rekrutierungsreihenfolge der Muskelfasern nicht eindeutig festgelegt zu sein und somit global zu erfolgen. Wird demnach ein einzelner Beckenbodenmuskel elektrisch oder durch eine rPMS stimuliert, besteht die Tendenz, dass sich auch die weiteren Muskelgruppen der Verbundeinheit kontrahieren, d.h. wie ein einziger Muskel reagieren. Wobei sich mit der Anzahl der durch ein Magnetfeld erfassten motorischen Einheiten die Wahrscheinlichkeit für eine Massenkontraktion erhöht. Dies hat eine weitreichende Bedeutung, denn es weist darauf hin" dass sich unterschiedliche Muskeln des Beckenbodens wie ein einziger Muskel verhalten, d.h. sich insgesamt kontrahieren (Massenkontraktion) und insgesamt entspannen. Was insoweit einem natürlichen physiologischen Ablauf entspricht, nachdem der innere (glattmuskuläre) und äußere (quergestreifte) Rhabdospinkter) keine ausreichende Kraft entwickeln, um einem erhöhten Druckanstieg im Bauchraum standzuhalten. Ein Kontinenzerhalt erfordert deshalb immer eine globale Muskelkontraktion des Beckenbodens, weil durch die damit entstehenden divergierenden Zugrichtungen des Beckenbodens, nämlich nach vorne oben und nach hinten unten, die Harnröhre hochgezogen wird ("Integraltheorie nach Petros") und durch die dabei entstehende "Abknickung" ein effektiver Reserveverschluss entsteht.

Was nun einer perfekten Anforderung einer Kontinenz-Wiederherstellung entspricht, ignoriert die bei den weiteren rPMS-Einsatzgebieten (sexuellen Dysfunktion / Post-Partum-Training / Inkontinenz nach Prostatektomie / Stuhlinkontinenz / Schambeinentzündung ) notwendige Ansteuerung spezieller, funktionsbezogener Muskeln und Sehnenansatzpunkte, die fokussiert und verstärkt zu trainieren sind.

Auch macht es die schnelle Ermüdbarkeit extensiv trainierter Muskelfasern erforderlich, während einer rPMS-Therapiesitzung Entlastungen bzw. Pausen vorzusehen. So gilt als Grundregel der Trainingslehre, dass die Gesamtsumme der Reize nicht länger sein sollte als die Pausenzeit. Ist aber die Pausenzeit zu lang, verblasst die Anpassungsreaktion (Adaption auf zellulärer Ebene), d.h. bleibt der Reiz nur unterschwellig. Werden mehrere große Muskelgruppen gleichzeitig aktiviert, sind vor allem die energetischen Ressourcen zu beachten. Da eine rPMS wesentlich intensiver wirkt als jedes freie, aktive Muskeltraining, kommt es sehr schnell zur Verarmung von Energiereserven, einer Zunahme von Laktat, die von einer Enzymhemmung durch Übersäuerung begleitet ist, Elektrolytverschiebungen sowie einer ungenügenden Freisetzung des Botenstoffs Acetylcholin. Dies bestätigt sich auch durch eine mit dem E-Puls durchgeführten Studie an der Bundeswehr-Hochschule München (Penka G. Pylypiw T. Elektromagnetische Stimulation der menschlichen Muskulatur. Universität der Bundeswehr München. Bericht vom 06.02.2010), nach der es - trotz Pausenschaltung - schon nach einer gewissen Behandlungszeit, zu einer Verschlechterung des bis dahin erreichten Trainingsergebnisses kommt.

Das Verfahren zur Magnetfeldstimulation von Körpergewebe eines Benutzers ist ferner dadurch gekennzeichnet, dass es unter der Zielsetzung einer "5-fach-Therapie" verläuft. Neben der Hauptfunktion eines Beckenboden-trainings sowie einer Reflexhemmung von Entleerungssignalen an den M. detrusor vesicae, können optional 4 weitere Behandlungstools - getrennt voneinander - zugeschaltet werden. Dies verbessert nicht nur das Therapieergebnis, sondern erlaubt weitere, über die die Inkontinenzbehandlung hinausreichende Effekte auf den Organismus.

Die Zusatzfeatures bestehen:
1. Aus dem Modul Telemedizin: Um dem Patienten einen höchstmöglichen therapeutischen Support zu bieten und um die Therapieeffekte noch weiter zu verstärken, wird die rPMS-Behandlung durch die exklusive Verfügbarkeit einer telemedizinischen Beratung begleitet. Diese kann von den Patienten - unabhängig der telemedizinischen Anfangs- und Endbefundung - in jeder Phase der Behandlung in Anspruch genommen werden und ist untrennbarer und wichtiger Bestandteil des E-Puls-Trainingskonzepts
2. Aus der Zuführung von Sauerstoff, welche über eine Atemmaske oder eine Nasenbrille erfolgt und in Kombination mit einer rPMS-bedingten Aktivierung großer Muskelbereiche (Beckenboden, Gluteus-, Hüft-, Oberschenkel- und untere Rückenmuskulatur) eine nachhaltige postkapilläre Endothelabschwellung zur Folge haben kann und einer nachhaltigen mikrozirkulatorischen Verbesserung dient.
3. Aus einer sich an die Hauptbehandlung anschließenden Frequenztherapie (PEMF), die über dezidierte Frequenzmodulationen von Delta-, Theta- und Alphawellen (1 - 12 Hz) zu einer thalamischen Reaktion und Veränderung der Hirnwellenaktivität und damit zur Entspannung der durch die rPMS entstandenen vegetativen Erregung führt.
4. Aus der optionalen Zuschaltung einer Lichttherapie. Diese dient nicht nur der Behandlung von bei Inkontinenzproblemen häufig auftretenden depressiven Episode (Grad 1 - 3), Major depressions oder auch einer saisonalen Depressionsymptomatik. Vielmehr wurde neuen Erkenntnissen zur Photobiomodulation, der therapeutischen Wirkung bestimmter Farbspektren sowie des Frequenzbereichs der Nahen Infrarot LED-Technik Rechnung getragen. Hieraus ergeben sich weitreichende Konsequenzen in der Prophylaxe und Therapie der Hautalterung bzw. Hautrejuvenation, in der Akne- und Psoriasisbehandlung, der Wundbehandlung, der Behandlung des androgenetischen Haarausfalls, der Schmerzbehandlung bei Arthrosen / Arthritiden und Ischialgien, Neuropathien bis hin zu Regeneration nach Nervenverletzungen, der Demenzbehandlung, sonstigen Schmerzen des Bewegungsapparats sowie der allgemeinen Entzündungsbehandlung.

Die hierfür eingesetzte Technik basiert auf Erkenntnissen der von der NASA (National Aeronautics and Space Administration) weiterentwickelten LED-Technik (Light Emitting Diodes), die nicht nur die blauen (450 - 490 nm) und roten (630 - 700 nm) Farbspektren bzw. Wellenlängenbereiche umfasst, sondern auch die "Nahe Infrarot LED" mit bis zu 880 nm. (Whelan HT, Houle JM, Whelan NT et al. The NASA Light-Emitting Diode Medical Program - Progress in space flight and terrestrial applications. 2000. DOI: 10.1063/1-1302454. Corpus ID: 14376705). Letztere ist in der Lage, über eine Besetzung farbsensitiver Cytochrome die mitochondriale Aktivität anzuregen. Denn das mitochondriale Chromophor Cytomchromoxidase c akzeptiert die Photoenergie bestimmter Wellenlängen, womit etwa 50 % des Nahen IR-Lichts zur Absorption gelangen. Eine Wellenlänge von 630 - 700 nm ist dem roten Farbspektrum zuzuordnen. Wobei hervorzuheben ist, dass rotes Licht nicht nur die DNA- und RNA-Syntheserate erhöht, sondern auch insgesamt 111 Gene mit 10 verschiedenen physiologischen Funktionen moduliert (Zang Y, Song S.,Fong C et al. cDNA microarrary analysis of gene expression proteins in human fibroblast cell after irradiation with red light. J. Invest. Dermatol. 2003; 120, 849-857).

Lichtphotonen der Wellenlängen 630 - 800 nm haben eine Tiefenreichweite von bis zu 23 cm und bezieht sich auf das "Optical Window" der Haut oder auch "Near IR window" des Körpers (Karu TI. Multiple roles of cytochrome c oxidase in mammalinan cells under action and IR-A radiation. IUBMB J. 2010; 62(8): 607-610)

Die Photobiomodulation hat die Synthese mitochondrial erzeugten ATPs (Adenosin-Tri-Phosphat) zur Folge. ATP kommt dabei nicht allein der Aufgabe als Energieträger zu. Vielmehr fungiert es auch als Signalmolekül, das Zellen und sonstiges Gewebe in die Lage versetzt, untereinander zu kommunizieren (.Burnstock G. Puriness and sensory nerves. Exp Pharmacol. 2009: 194: 332-392 // Kakh BC, Bumstock G. The double life of ATP. Sci Am. 2009; 12: 84-92).

So ist bekannt, dass Nervenzellen offensichtlich ATP-Boten in die Muskeln, den Darm und die Blase entsenden, die über sog. P2-Rezeptoren (Subtypen P2X und P2Y11) unterschiedliche zelluläre Effekte erzeugen können. So gibt es Studiennachweise, dass sich P2Y2- und P2Y11-Rezeptoren ausbilden, wenn humane neuronale Progenitorzellen einer Bestrahlung im Wellenlängenbereich von 810 nm unterliegen. Dies entspricht in seiner Wirksamkeit den Effekten körpereigener Wachstumsfaktoren. Die neue Rolle, die ATP damit zukommt und die mittels naher IR-LED initiiert werden kann, erklärt damit auch die Vielseitigkeit photobiomodulatorischer Effekte.

Zwar entstehen durch eine Photobiomodulation mittels naher IR-LED oder auch rotes Licht auch Oxidantien (ROS). Nur dienen sie der Gen-Transkription bzw. einer notwendigen RNA-Synthese aus DNA, was daraufhin der zellulären Reparatur zugutekommt. Hier scheinen vor allem die Wellenlängenbereiche von 770 und 880 nm besonders effektiv zu sein (Calderhead RG. The photobiological basics behind light-emitting diode (LED) phototherapy. Laser Therapy. 2007;16:97-108)

In einem Wellenlängenbereich zwischen 630 - 1100 nm reduzierten sich - zumindest im Tierversuch - die Beta-Amyloid-Ablagerungen bei einem künstlich herbeigeführten M. Alzheimer (Grillo SL, Duggett NA, Ennaceur A, Chazot PL. Non-invasive infra-red therapy (1072 nm) reduces beta-amyloid protein levels in the brain of an Alzheimer's disease mouse model, TASTPM. J Photochem Photobiol B. 2013;123:13-22, kam es bei M. Parkinson zu einem geringeren Untergang dopaminproduzierender Zellen, milderte sich die Symptomatik im transgenen Mäusemodell einer Demenz, führte zu einer verkürzten Regenerationszeit nach Nervenverletzungen, einer Schmerzreduzierung bei Ischialgie oder neuropathischen Schmerzen. In Humanversuchen kam es zu einer deutlichen Besserung einer Mucositis nach Chemotherapie und reduzierten sich die Schmerzen eines Karpaltunnel-Syndroms, Schmerzsyndrome des Kiefergelenks, Nackenschmerzen oder neuropathische Schmerzen nach Amputation.

Ausschlaggebend hierfür ist eine nahe IR-LED bedingte Herunterregelung proentzündlicher Cytokine wie IL-6, MCP-1, IL-1ß, NFF-alpha, wie sich unter den Wellenlängen 633, 780, 810 und 950 nm nachweisen lässt (Fernandes KP, Souza NH, Mesquita-Ferrari RA et al. Photobiomodulation with 660-nm and 780-nm laser on activated J774 macrophage-like cells: effect on M1 inflammatory markers. J Photochem Photobiol B. 2015; 153:344-351)..

Auch Leistungs- und Spitzensportler profitieren im Verletzungsfalle von einer Photobiomodulation, da man damit die Rekonvaleszenz erheblich verkürzen kann. So reichten in einer Studie lediglich 2 - 6 Behandlungen mit einer Wellenlänge von 830 nm aus, um die Rehabilitationszeit von 19 Tagen auf weniger als 10 Tage zu verkürzen (Foley J, Vasily DV, Bradle J et al. 830 nm light-emitting diode (led) phototherapy significantly reduced return-to-play in injured university athletes: a pilot study. Laser Ther. 2016; 25(1): 35-42

Im Falle eines androgenetischen Haarausfalls erhöhte sich z.B. mittels 16-wöchiger IR-LED- Behandlung die Haardichte gegenüber Placebo um 35 % (Lanzafame RJ, Blanche RR, Bodian AB et al. The growth of human scalp hair mediated by visible red light laser and LED sources in males. Lasers Surg Med. 2013; 45(8): 487-495).

Besonders hervorzuheben sind auch die Therapieeffekte der nahen IR-LED auf die Konsistenz bzw. dem Alterungsprozess der Haut. Diesem liegt in der Regel eine Downregulation der Kollagensynthese bei gleichzeitiger Zunahme der Matrix-Metalloproteinasen MMP, welche für den Gewebeabbau verantwortlich sind, zugrunde. Mit einer LED-Behandlung des Wellenlängenbereich von 660 nm konnte z.B, der Kollagenabbau um 31 % erhöht und die MPP-Zunahme um 18 % reduziert werden. Wellenlängen von 633 und 830 nm wiederum führten zu einer Faltenrückbildung um bis zu 36 % und einer Verbesserung der Hautelastizität um bis zu 19 %, was einer erheblichen Rejuvenation der Haut entspricht. Dabei beschränken sich die Therapieeffekte von rotem Licht bzw. einer Low Level Light Therapie (LLLT) nicht nur auf den Dermisbereich, sondern erstrecken sich auch auf die Epidermis. Auch bei Hautverbrennungen 3. Grades kam es im Tierversuch bei in den Bereichen 660 und 780 nm zu einer deutlichen Kollagensynthese und auch Kollagenreifung, wobei unter grünem Licht (520 - 550 nm) ähnliche, wenn auch nicht ganz so starke Therapieeffekte nachzuweisen waren.

Neben den roten und IR-Spektren kommt auch dem rein blauen Licht (450 - 490 nm) eine besondere Bedeutung zu. Grundsätzlich wirkt helles Licht stark auf den menschlichen zirkadianen Rhythmus ein, weil es die Melatoninsynthese hemmt (460 - 480 nm / 446 - 477 nm) und somit die Wachheit und Vigilanz (Aufmerksamkeit) steigern kann. Besonders stark wirkt hier die Wellenlänge von 464 nm ("himmelblau"), indem sie den Sinnen Tagesbedingungen signalisiert. Mit zunehmendem Alter nimmt die Blaulicht-Sensitivität für diese NIH-Faktoren wie z.B. einer Kognitionsverbesserung zwar ab. Andererseits scheint blaues Licht gerade bei älteren Patienten mit einem dementiellen Syndrom (ADRD) bzw. M. Alzheimer günstig auf den Schlaf, die Agitationen und Depressionen einzuwirken.

Blaues Licht ist der Schlüsselreiz für die Zirbeldrüse, was die Produktion von Melantonin stoppt. Folgerichtig kommt blaues Licht bereits in bestimmten Flugzeugtypen (A350 Airbus / 757 Boeing) zum Einsatz, weil es den Jetlag bei Flügen von West nach Ost weitgehend unterbinden kann (Leder A. Komfortgewinn für Passagiere auf Langstreckenflügen. Springer Fachmedien Wiesbaden. 2016)

Rotes Licht (630 - 700 nm) dagegen scheint die Produktion von Melatonin zu fördern. Unter Rotlicht verbesserte sich damit nach etwa 14 Tagen nicht nur der Serum-Melatoninspiegel sowie die Schlafqualität, sondern auch die Ausdauer von weiblichen Basketballspielerinnen (Zhao J, Tian Y, Nie J et al. Red light and the sleep quality and endurance performance of chinese female basketball players. J Athl Train. 2012; 47(6): 673-678)

Ein weiterer Meilenstein in der Lichtspektrenforschung ist die Entdeckung des Melanopsin-Rezeptors im Auge, also einem Photorezeptor, der dem retinohypothalamischen Pfad im Gehirn entspricht. Diese Bahnen werden auch als sog. "Non-Imaging-Forming Factors (NIF) bezeichnet, weil sie nicht über den Sehtrakt verlaufen und somit auch nicht mit visuellen Effekten verbunden sind. Mit diesem neuronalen Signalpfad sind endokrine (hormonelle) sowie neurobiologische Verhaltenseffekte verbunden, die sich auf die Körpertemperatur, Cortisolsekretion, Herz-Frequenz, Aufmerksamkeit, kognitive und psychomotorische Performance, die Hirndurchblutung, das Gedächtnis und Gen-Expression auszuwirken scheinen. So verringern sich unter blauem Licht verstärkt die Alpha- Theta- und Deltawellen, was eine wesentlich schnellere Reaktionszeit zur Folge hat und ansonsten allfällige Aufmerksamkeitslücken reduzieren.

Blaues Licht führt generell zu einer "entspannten" Aufmerksamkeit, womit sich auch die Geschwindigkeit der Informationsverarbeitung verbessert (Lehrl S, Gerstmeyer K, Jacob JH et al. Blue light improves cognitive performances. J Neural Transm. 2007; 114: 457-460). Bei leuchtenden , Blauspektren zeigten sich Studenten in Lernsituationen wesentlich entspannter als unter anderen Farben. Während sich in potentiellen Stresssituation unter gelb oder rot die Herzfrequenz erhöht, bleibt sie unter blauem Licht unverändert.

Besonders interessant ist das Ergebnis von Studien, nach denen es unter blauem Licht zu einer signifikanten Syntheseverstärkung des Luteinisierenden Hormons (LH) in der Hypophyse kommt. Bekanntlich führt dies beim Manne zu einer Verstärkung der Testosteronproduktion in den Leydig-Zellen der Hoden bzw. bei der Frau zu mehr Östrogen (Kripke DF, Elliott JA, Youngstedt SD et al. Weak evidence of bright light effects on human LH and FSH. J Circadian Rhyhtms. 2010; 8: 5). Auch ist damit ein Anstieg des follikelstimulierenden Hormons FSH verbunden (Danilenko K, Sergeeva OY. Immediate effect of blue-enhanced light on reproductive hormones in women. Neuroendrinol Lett. 2015; 36(1): 84-90) Neben den kognitionsfördernden Aspekten von blauem Licht, steht auch die Therapie bestimmter Hauterkrankungen wie die Akne vulgaris und der Psoriasis im Vordergrund. Die Akne entsteht durch eine hormonell bedingte Steigerung der Talgproduktion, wobei sich die Ausführungsgänge verstopfen und es über die darin befindlichen anaeroben Proprionibakterien zu Entzündungen kommt. Blaues Licht besitzt hier ein beachtliches antimikrobielles Potential und entwickelt damit auch einen förderlichen Einfluss auf das Mikrobiom der Haut. Auch Infrarot-Laser mit den Wellenlängen von 1320, 1450 und 1540 nm üben auf die talgproduzierenden Zellen einen direkten phototoxischen Effekt aus und verringern damit die Talgproduktion. In einem systematischen Review über insgesamt 25 randomisierte und placebokontrollierte Studien zeigte sich, dass z.B. blaues Licht, blau-rotes Licht oder auch Infrarot eine hohe Erfolgswahrscheinlichkeit in der Behandlung einer Akne besitzen.

In einem Wellenlängenbereich von 405 - 420 nm reduzierte sich z.B. die Anzahl der Aknepusteln um 60 - 70 %, wenn über einen Zeitraum von 4 Wochen (2 x wöchentlich) bestrahlt wurde. Mit einer Kombination aus blauem und roten Licht nahm die Pustelanzahl um 69 - 77 % ab (Hamilton FL, Car J, Lyons C et al. Laser and other light therapies for the treatment of acne vulgaris: Systematic review. Br J Dermatol. 2009; 160:1273-1285). Ähnlich lagen die Ergebnisse einer jüngeren Doppelblindstudie, die Blau-Rot mit Placebo verglich und nach einer Behandlungszeit von 2 x täglich über 2,5 Minuten über einen Zeitraum von 4 Wochen eine Aknereduktion von 77 % (entzündet) und 54 % bei nicht entzündeten Pustel erbrachte.

Vergleicht man z.B. die topische Anwendung des Antibiotikums Clindamycin mit der Applikation von blauem Licht, lag die Blaulichtbehandlung mit einer Akne-Reduktionsrate von 34 % gegenüber Clindamycin mit 14 % klar im Vorteil. In einem weiteren Literaturreview zu verfügbaren Studien, in den LEDs der Wellenlängen 415 nm (blau), 633 nm (rot) und 830 nm (Infrarot) zum Einsatz kamen, ergaben sich signifikante Therapieeffekte bei der Akne vulgaris, Wundheilung, Aktinischen Keratose, einem Basazell-Karzinom und kosmetischrejuvenativer Einsatzgebiete (Ablon G. Phototherapy with light emitting diodes. J Clin Aesthet Dermatiol. 2018; 11(2): 21-27).

Ein weiteres Einsatzgebiet für Blaulicht ist die Psoriasis. In einer randomisierten Doppelblindstudie, in der die Therapieeffekte von blauem (420 - 453 nm) und rotem Licht verglichen wurden (3 x wöchentlich über 4 Wochen), war das blaue Licht wirksamer als der rote Wellenlängenbereich.

Blaues, rotes Licht, aber auch die nahe IR-LED, werden sehr gut vertragen, da sie - anders als z.B. Laser - weder die Haut noch sonstiges Gewebe schädigen (Pei S, Inamadar AC, Adya KA et al. Light-based therapies in acne treatment. Indian Dermatol Online J. 2015; 6(3): 145-157). Insgesamt gehören LED-basierte Lichtsysteme zu einer der sichersten Tools in der Behandlung entzündlicher Erkrankungen der Haut, deren Alterung (Sorbellini E, Rucco M, Rinaldi F et al. Photodynamic and photobiological effects of light-emitting diode (LED) therapy in dermatological disease: an update. Lasers Med Sci. 2018; 33(7): 1431-1439), der mitochondrialen Energiegewinnung, der Hormonstimulation, der Steigerung von Vigilanz oder der Förderung des Haarwachstums.

In dem im E-Puls eingesetzten Lichttherapiesystemen sind die einzelnen Wellenlängenbereiche für blaues, rotes und nahe IR-LED exakt einstellbar und erlauben damit eine gezielte Therapie entsprechend des jeweiligen Einsatzgebiets..

Durch die oben angegebenen Zusatzmaßnahmen kann die Therapiewirkung der Magnetfeldstimulation deutlich erhöht werden gegenüber einer Standard-Magnetfeldstimulation gemäß dem Stand der Technik.

Nachfolgend wird ein bevorzugtes Ausführungsform der Erfindung anhand der Zeichnungsfiguren beschrieben. Aus den Zeichnungen und ihrer Beschreibung ergeben sich weitere Merkmale und Vorteile der Erfindung.
- Figur 1: zeigt eine perspektivische Seitenansicht der Magnetfeldstimulationsvorrichtung.
- Figur 2: zeigt eine perspektivische Frontansicht der Magnetfeldstimulationsvorrichtung.
- Figur 3A: zeigt schematisch die Stärke (Energie) des Magnetfelds über die Zeit bei der erfindungsgemäßen Vorrichtung zur Magnetfeldstimulation
- Figur 3B: zeigt schematisch die Stärke (Energie) des Magnetfeldes über die Zeit bei einer Magnetfeldstimulationsvorrichtung gemäß dem Stand der Technik.
- Figur 4A: zeigt schematisch die magnetische Flussdichte des Magnetfelds über die Zeit bei der erfindungsgemäßen Vorrichtung zur Magnetfeldstimulation
- Figur 4B: zeigt schematisch die magnetische Flussdichte des Magnetfeldes über die Zeit bei einer Magnetfeldstimulationsvorrichtung gemäß dem Stand der Technik.
- Figur 5A: zeigt schematisch die Wirkfläche des Magnetfelds der erfindungsgemäßen Vorrichtung zur Magnetfeldstimulation.
- Figur 5B: zeigt schematisch die typische Wirkfläche des Magnetfelds bei einer Magnetfeldstimulationsvorrichtung gemäß dem Stand der Technik.
- Figur 6: zeigt schematisch eine in Pfeilrichtungen verschiebbare Magnetspule unterhalb der Sitzfläche der Vorrichtung.
- Figur 7: verdeutlicht schematisch die effektivere Wirkung der erfindungsgemäßen Vorrichtung zur Magnetfeldstimulation auf das Körpergewebe eines Benutzers im Vergleich zum Stand der Technik.

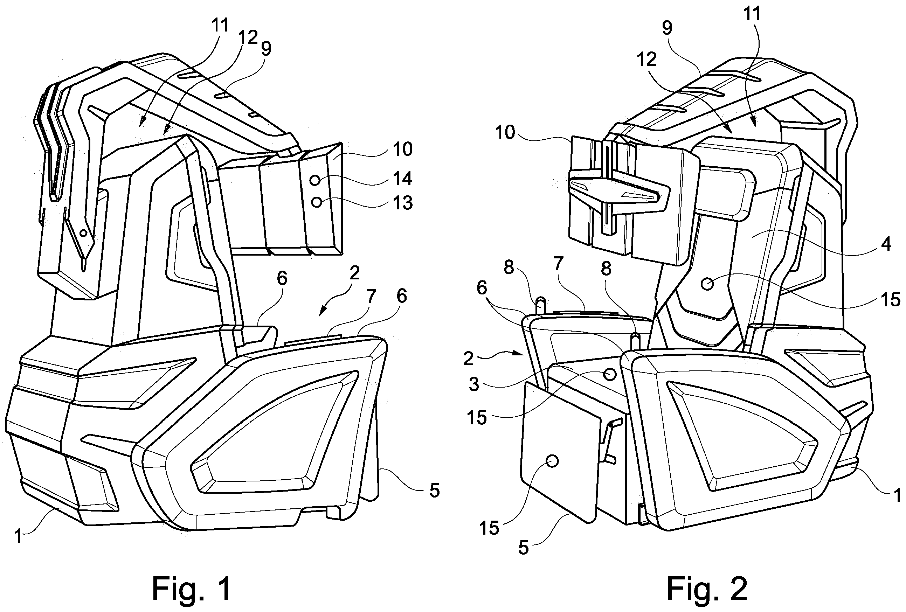

Die Vorrichtung zur Magnetfeldstimulation gemäß den Figuren 1 und 2 umfasst ein Grundgestell 1, auf welchem eine Sitzgelegenheit beispielsweise in Form eines bequemen einstellbaren Sessels 2 montiert ist. Das Design der erfindungsgemäßen Vorrichtung zur Magnetfeldstimulation spielt für die Funktion der Erfindung keine Rolle und soll hier nur beispielshaft dargestellt sein. Ferner sind vorhandene technische Einrichtungen teilweise nur schematisch dargestellt.

Die Sitzgelegenheit 2 umfasst eine Sitzfläche 3, eine Rückenlehne 4 und ein Fußteil 5, die verstellbar sind, so dass der Benutzer eine für ihn angenehme Sitz- oder Liegeposition einnehmen kann. Die Verstellung der Sitzgelegenheit 2 erfolgt über ein Bedienpult 7 manuell oder automatisch anhand einer zuvor vom Benutzer gespeicherten und wieder abrufbaren Sitz- oder Liegeposition. Ferner umfasst die Sitzgelegenheit 1 eine Armlehnen 6, wobei an einer oder beiden Armlehnen 6 das Bedienpult 7 angeordnet ist. Das Bedienpult 7 ist mit einer Tastatur und/oder einem Touchscreen ausgestattet.

Das Gerät verfügt vorzugsweise über eine Lichthaube 9. In oder an der Lichthaube 9 ist ein Infotainmentsystem zum Abspielen von Musik, Ton- und Klangeffekten sowie Lichteffekten vorgesehen. Das Infotainmentsystem umfasst mindestens einem integrierten Bildschirm 10 im Sichtbereich des Benutzers, einem Mikrophon 13, Lautsprecher 11 bzw. einen Bluetooth-Kopfhörer mit integriertem Mikrofon und Beleuchtungsmittel 12. Es kann ebenfalls eine auf den Benutzer gerichtete Kamera 14 vorgesehen sein. Diese Einrichtungen dienen nicht nur der Kommunikation mit einem (externen) telemedizinischen Zentrum, sondern auch der unterstützenden Behandlungsinformation des Benutzers, korrekten Durchführung eines "High-Mental-Effort-Trainings" sowie als themenübergreifendes Infotainmentsystem.

Die Vorrichtung zur Magnetfeldstimulation umfasst vorzugsweise mehrere Funktionsmodule, welche die verschiedenen Funktionen der Vorrichtung ermöglichen. Die Funktionsmodule können von dem Benutzer angewählt werden und unterstützen, ergänzen und verbessern eine Behandlung des Benutzers mit dem Magnetfeldstimulationsgerät. Die Funktionsmodule des Magnetfeldstimulationsgeräts stehen über die Einrichtungen des Infotainmentsystems, insbesondere Bedienpult 7, Bildschirm 10, Lautsprecher 10, Mikrofon 13 und Kamera in Verbindung und/oder Dialog mit dem Benutzer und der Zentrale.

### Modul Infotainment

Dieses Modul der Information und Unterhaltung richtet sich bevorzugt an rPMS-Anwender, für die ein High-Mental-Effort-Training nicht in Frage kommt. Da das Training bis zu 20 Minuten dauert, wird diese Zeit in den Folgesitzungen dazu genutzt, den Benutzer mit Informationen rund um die Harn- oder Stuhlinkontinenz, der Sarkopenie und Sturzgefahr oder Fakten zur Leistungssteigerung zu versorgen. Gerade die Sarkopenie, deren Muskelmasseverlust bereits in der 4. Lebensdekade beginnt und sich zwischen dem 50. und 80. Lebensjahr auf bis zu 3 % jährlich beschleunigt und der vor allem ein Abbau der Alpha-Motoneurone des Spinalganglions, aber auch die Abkopplung motorischer Einheiten oder die Infiltration von Binde- und Fettgewebe zugrunde liegt, ist ein ernstzunehmendes Alterungsphänomen. Die Abnahme der Skelettmuskelkraft betrifft hauptsächlich nur den Abbau schneller Muskelfasern (Fast-Twitch-Fibers). So wirkt sich eine Abnahme von Muskelmasse und Muskelkraft besonders auf die unteren Extremitäten aus, was umso schwerer wiegt, als dass das Gehen zu den essentiellen Notwendigkeiten des Alltags zählt und auch den Core tangiert. Denn diese Kraftverteilungsbox ist nicht nur das stabilisierende Element für sämtliche körperlichen Aktionen, sondern ist auch für das Gleichgewicht und die Balancefähigkeit essentiell. Bei einem unsicherer Gang steigt jedoch die Sturzgefahr und damit auch das Mortalitätsrisiko (Freiberger E, Sieber C, Pfeifer K. Physical activity, exercise, and sarcopenia- future challenges. Wien Med Wochenschr. 2011; 161(17-18): 416-425). So sind Stürze und Frakturen die häufigste Todesursache bei Menschen über 65 Jahren bzw. bedingen häufig eine spätere Heimeinweisung, was umso bedenklicher ist, als dass jährlich allein 25,7 % aller Frauen zwischen 65-79 Jahren wegen eines Sturzes ärztlich behandelt werden müssen.

Die Bedeutung einer rPMS besteht deshalb nicht nur in der Behandlung einer Harninkontinenz, eines myofaszialen Schmerzsyndroms, einer sexuellen Dysfunktion oder in der Verbesserung einer sportlichen Performance. Vielmehr bietet sie die Möglichkeit, dem altersbedingten Muskelabbau der dominanten Gehmuskulatur und die mit diesem verbundene Störung der Balancefähigkeit ohne eigenen Kraftaufwand aufzuhalten.

Mit dem Infotainment-System wird der Anwender (Benutzer / Sportler) aber auch über den Zusatznutzen spezifischer Substrate informiert, die sich auf die Trainingsphysiologie und den optimalen Nährstoffbedarf eines intensiven Muskeltrainings bezieht. So gibt es Erkenntnisse zur Leistungsverbesserung durch Säurepufferung bei den hier vorliegenden laktaziden Belastungen von kurzer Dauer oder auch repetitiven hochintensiven Belastungen durch die Supplementierung eines bestimmten Dipeptids. Auch dürfte es einen Anwender interessieren, wie z.B. der Einsatz von sog. BCAA (Branched-Chain Amino Acids) bei dem mit dem rPMS hier simulierten "Widerstandstraining" zu werten ist, oder welche Bedeutung Pausenzeiten während der rMPS-Behandlung besitzen.

Fokussiert sich eine rPMS vorrangig auf den Fettabbau (z.B. auch das viszerale Fett), ist es für den Anwender z.B. wichtig zu erfahren, wie über eine mögliche Aktivierung von sog. PPARa (Peroxisome Proliferator Activated Receptor alpha) die Fettverbrennung durch die Leber forciert werden kann. Geht es bei der rPMS um eine die erektile Dysfunktion (Impotenz), sollte - anders als bei der weiblichen sexuellen Dysfunktion - auf animierte sexuelle Filminhalte verzichtet werden.

Das Infotainment beschränkt sich aber nicht nur auf gesundheitsbezogene Informationen, sondern liefert auch andere Informationen, z.B. Hinweise zum Wetter, Wirtschaftsnachrichten und Breaking-News / Eilmeldungen.

### Gesichtserkennung

Die erfindungsgemäße Magnetfeldstimulationsvorrichtung ist via Bildschirm 10 und Kamera 14 mit einer Gesichtserkennungssoftware ausgestattet, durch welche dem Behandler die notwendigen Benutzerdaten sowie Namen und Zusammenhänge aus früheren Gesprächen (z.B. Lieblingsgetränk, Kaffee mit oder ohne Milch und Zucker, Name der Kinder, deren bevorstehende Klassenarbeit, Heirat , Name des Hundes, der Katze etc.) zu Beginn des Benutzer-Settings im Display zur Verfügung stehen. Die Gesichtserkennung bzw. die KI bietet auch die Möglichkeit, den Benutzer auf Basis einer Sprachsoftware persönlich zu begrüßen, sich nach dessen Wohlbefinden zu erkundigen oder ihn auf sein gutes Aussehen anzusprechen etc. Auch besteht die Verabschiedung durch die Sprachsoftware nicht aus den üblichen Floskeln, sondern aus einem individuellen wortwitzigen Verabschiedungsgruß - immer abhängig vom Krankheitsbild, der Schmerzsituation, dem Grund der sexuellen Dysfunktion oder sportlichen Ambitionen.

Hintergrund dieser persönlichen Ansprache des Benutzers durch die Vorrichtung ist die hohe Prävalenz einer Inkontinenz in Verbindung mit einer depressiven Symptomatik (20,6% - 43,0 %) (Avery J, Stocks N. Urinary incontinence, depression, and psychosocial factors - a review of population studies. EMJ. 2016; 1(1): 58-67), die durch eine persönliche Ansprache und einer damit verbunden "Zuwendung" beeinflusst werden kann.

### Soundsystem

In der Lichthaube 9 der Vorrichtung ist beidseits ein Sound-System mit Lautsprechern 11 verbaut, das nicht nur ein einzigartiges Ton- und Klangerlebnis bietet, sondern auch dazu genutzt wird, nach der Gesichts- bzw. Benutzererkennung ein präferiertes Musikstück des Benutzer in einer kurzen Sequenz anzuspielen. Oder es eröffnet als Stimmungsmittel die Möglichkeit, aus einem Mix aus tiefen vibrierenden Tönen eine Behandlungsdramaturgie aufzubauen.

### Bedienpult / Touchscreen

Das Bedienpult 7 mit Touchscreen besteht aus einem schwenkbaren (360 Grad), senkrecht auf der rechten Armlehne der Vorrichtung platzierten Bildschirm, der es nicht nur dem Therapeuten erlaubt, den Behandlungsablauf zu programmieren, sondern auch dem Benutzer die Möglichkeit bietet, die Behandlung per Fingertip zu unterbrechen oder die Restzeit der Behandlung abzufragen. Er enthält auch einen "Notfall-Button", mit dem bei Unpässlichkeit des Benutzers oder sonstigen unerwünschten Wirkungen, das therapeutische Assistenzpersonal benachrichtigt werden kann. Alternativ oder ergänzend zum Touchscreen kann eine Eingabetastatur vorgesehen sein.

Je nachdem, ob die Vorrichtung durch einen Arzt bzw. ein ärztlich geleitetes Behandlungszentrum oder durch einen Physiotherapeuten auf der Basis einer ärztliche Verordnung betrieben wird und es sich nicht lediglich um ein rein sportliches oder prophylaktisches Beckenbodentraining handelt, entscheidet das Touchscreen-Programm über die datenschutzrechtliche Zugangsberechtigung zum Abruf oder elektronischen Weiterleitung dort gespeicherter Benutzer- und Behandlungsdaten. Dies geschieht durch Eingabe eines elektronischen Schlüssels, der erst nach vorheriger Einverständniserklärung des Benutzers zur Verfügung steht.

### Modul Telemedizin

Die Vorrichtung ist insbesondere geeignet für die Verwendung in der digitalen Telemedizin. Ein in der Erstsitzung des Benutzers von der Vorrichtung konsultiertes telemedizinisches Zentrum erhebt mittels eines digitalen, auf die Befundkonstellation (Urologie / Gynäkologie) abgestimmten Fragebogens (Tabletversion) eine Anamnese des Benutzers, die von einer Software mit bereits erhobenen Daten anderer, früherer Benutzer abgeglichen wird. Bei ungewöhnlichen Befundkonstellationen signalisiert das System weiteren Nachfragebedarf und überprüft bisherige Verordnungsentscheidungen auf Konformität oder Kollision zur eigenen Behandlungsempfehlung. Bezieht sich die Behandlungsempfehlung auf ein gerätetechnisches Beckenbodentraining mittels repetitiver peripherer Magnetfeldstimulation (rPMS), wird der Benutzer über eine digitalisierte Einverständniserklärung zu möglichen Kontraindikationen und Nebenwirkungen der Behandlung informiert. Ähnliches gilt auch für eine Entbindung von der ärztlichen Schweigepflicht, damit der Betreiber des physikalischen Beckenbodentrainings die entsprechenden Geräteeinstellungen vornehmen kann. Unabhängig davon hat der Benutzer die Möglichkeit, sich jederzeit von einem Arzt zu wichtigen Behandlungsfragen oder unerwarteten Nebenwirkungen telemedizinisch beraten zu lassen.

### Modul Behandlungsinformation

Das Modul Behandlungsinformation, das für die ersten Behandlungssitzungen zur Verfügung steht, setzt sich aus einer Vielzahl von Animationssequenzen zusammen, die sich auf die einzelnen, mit der Vorrichtung behandelbaren Indikationen beziehen und die die meist komplexen urologischen und gynäkologischen Veränderungen und Defizite des Beckenbodens, der Blase und des Blasenverschlusssystems anschaulich und prägnant erklären. Diese werden dem Benutzer mit dem Infotainmentsystem präsentiert. Darauf aufbauend soll der Benutzer - fast zeitgleich mit den realen Aktionen der repetitiven peripheren Magnetfeldstimulation (rPMS) - nachvollziehen können, nach welchem Wirkprinzip die Behandlung verläuft und worauf die Heilung oder Remission der Erkrankung basiert.

So lernt die Benutzerin mit einer Belastungsinkontinenz, wie z.B. eine Steigerung der Muskelkraft des Beckenbodens die Harnröhre natürlicherweise in Richtung Schambein zieht ("Integraltheorie nach Petros"), woraus - vergleichbar einer chirurgischen Inkontinenzbehandlung mittels TVT/TOT - ein natürlicher Knickverschluss der Harnröhre entsteht, wobei das durch Muskelkontraktionen aktivierte Repräsentationszentrum des Zentralen Nervensystems (ZNS) die Nachhaltigkeit des Behandlungsergebnisses verstärkt. Benutzer mit einer überaktiven Blase (OAB-Syndrom) oder einer Dranginkontinenz ist es wiederum möglich nachzuvollziehen, wie sich der Reiz- und Entleerungszustand der Blase durch Blockierung von Leitungsbahnen des Entleerungszentrums im ZNS herunterregeln lässt. Oder es lernen Inkontinenz-Benutzer, die sich einer radikalen Prostatektomie unterzogen haben, wie wichtig es ist, den Beckenboden mithilfe einer rPMS in das OP-geschädigte Blasen-Verschlusssystem zu integrieren und damit das Defizit zu kompensieren. Benutzer mit einer erektilen Dysfunktion (Impotenz) wird die Bedeutung des "Kontinenzmuskels" M. ischiocavernosus näher gebracht und warum sich mit dessen Verstärkung eine penile venöse Abflussstörung ("venöses Leck") unterbinden lässt. Und Benutzer mit Hüftarthrose, einem Pelvic-Pain-Syndrom, einer Schambeinentzündung oder lumbalen Rückenschmerzen / Bandscheibengeschehen lernen, wie eine rPMSbedingte Auflösung des meist myofaszialen Hintergrunds, zu einer deutlichen Schmerzreduzierung führen kann.

Die sexuelle Dysfunktion zählt mit einer Prävalenz von 40 - 45 % zu den häufigen, jedoch oft nicht ernst genommenen Erkrankungen der Frau. Hier werden vor allem "fehlendes Verlangen" (64 %) und die "Anorgasmie" (35 %) genannt, gefolgt von Problemen bei der sexuellen Erregung sowie Schmerzen beim GV. Obwohl Partnerprobleme, sozialen Bedingungen, Entbindungsfolgen oder ein Östrogendefizit in der Menopause eine nicht unbedeutende Rolle spielen, scheint hauptsächlich ein "starker Beckenboden" für die Lust und die Orgasmusfähigkeit essentiell zu sein. Hier stehen vor allem die Mm. pubococcygeus (MPC) und ileococcygeus (MICC) im Vordergrund, nachdem diese auch für das vaginale Lustempfinden und die rhythmischen Kontraktionen beim Orgasmus verantwortlich sind. Durch ein rPMS-Training der Mm. pubo- und ileococcygeus wird Frauen mit Orgasmusproblemen bewusst, wie wichtig diese Beckenbodenmuskel für ihren Klimax ist.

Es gibt Hinweise, dass ein Beckenbodentraining (Kegeltraining) beim anorgasmatischen Syndrom ein besseres Ergebnis bringt, wenn Frauen dazu angehalten werden, sich während des Trainings subjektiven Gedanken sexuellen Inhalts hinzugeben. Dementsprechend sollte ein rPMS-Training der Mm. pubococcygeus und ileococcygeus nicht mit nüchternen Informationen zu diesen Muskeln begleitet werden, sondern mit animierten Sexualsequenzen, die im Abgleich mit sexualtherapeutischen Erkenntnissen zu von Frauen favorisierten Imaginationen zu erstellen sind.

### Modul Trainingsinformation

Der Core bzw. der Core-Strength gehört zu den bedeutendsten Erkenntnissen der aktuellen Sportmedizin und Trainingsmethodologie (Hibbs A, Thompson KG, French DN et al. Optimizing Performance by Improving Core Stability and Core Strength. Sports Med. 2008; 38(12): 995-1008). Denn die Kraftübertragung zwischen den unteren und den oberen Extremitäten oder die damit zusammenhängende Technik und Präzision einer Schlag-, Wurf- oder Kickbewegung ist untrennbar mit dem austarierten Gleichgewicht eine fiktiven "muskulären Box" des Beckens und dem Körperstamms verbunden ("Proximal stability for distal mobility"). Der Core setzt sich aus dem Zwerchfell, den Bauchmuskeln, der tiefen unteren Rückenmuskulatur sowie dem mehrschichtigen muskulären System des Beckenbodens zusammen. Während jedoch für die Bauch-, Becken-, Zwerchfell- und tiefen Rückenmuskeln eine Vielzahl von Trainingsmöglichkeiten zur Verfügung stehen, können Übungsansätze wie Pilates, Yoga, oder der "Gummiband assistierte Watschelgang" den eigentlichen Beckenboden nur unzureichend trainieren. So erfahren zwar die Körperstamm- und Beckenmuskeln meist einen beeindruckenden Performance-Gewinn, nur kommt es dabei durch einen nur unzureichend ausgebildete Beckenboden zu einem gefährlichen Ungleichgewicht in der muskulären Balance- und Koordinations-fähigkeit sowie zu ungewollten Technikdefiziten und Überlastungen in der ausführenden distalen Muskulatur. Das eigentliche Ziel, die Verletzungshäufigkeit in den Sportarten Soccer, Football, Rugby, Volleyball, Volleyball, Golf, Tennis, Hockey, Laufsportarten usw. zu senken, lässt sich damit nur schwer erreichen, da nur ein voll ausgebildeter Core in der Lage ist, den in den Extremitäten einzusetzenden Kraftaufwand auf ein Minimum zu reduzieren(Leetun DT et al. Core stability measures as risk factors for lower extremity injury in athletes. Med Sci Sports Exerc 2004: 36: 926-934).

So lassen sich einem Sportler, egal ob aus dem Leistungs-, Vereins- oder ambitionierten Breitensport, mittels animierter Bilddarstellung nicht nur die myofazialen Ketten sichtbar machen, sondern warum die Kraftübertragung aus den Extremitäten oder dem Körperstamm nur über das Zentrum (Core-Strength) erfolgen kann. Und wie mit einem rPMS-bedingter Kraft- und Koordinationszuwachs des Cores eine Optimierung der Kraftübertragung zwischen der unteren und oberen Extremität gelingt. Nur so ist es möglich, dass durch ein 8-wöchiges Core-Strength-Training die Verletzungsanfälligkeit um 42 % bzw. die Rehabilitationszeit nach einer Verletzung um 62 % sinken kann (Peate WF, Bates G, Lunda K et al. Core strength: A new model for injury prediction and prevention. J Occupat Med Toxicol 2007: 2: 3).

### Modul High-Mental-Effort-Training

Inzwischen ist es durch eine Vielzahl von Studien belegt, dass mentales Training nicht nur zu einer besseren Leistung führt (Feltz DL, Landers DM. The effects of mental practice on motor skill learning and performance: a meta-analysis. J Sports Psychol. 1983; 5: 25-27), sondern bei konzentrierter Visualisierung auch einen deutlichen Muskelzuwachs initiieren kann (Corbin CB. Mental practice. In: Morgan MP. (Ed.). Ergogenic aids and muscular performance. 1972: pp. 93-118: New York, Academic Press ). So gelang es durch ein rein mentales 12-wöchiges Training (15 Minuten/tgl.), die Muskelkraft um bis zu 35 % zu erhöhen (Ranganathan VK, Siemionow V, Liu JZ et al. From mental power to musclepower - gaining strength by using mind. Neurophysiologia. 2004; 42(7): 944-956). In der Folge vergrößert sich damit auch die entsprechende kortikale Repräsentation im Gyrus praecentralis des somatosensorischen Cortex (Hirnrinde), also dem Repräsentationszentrum für Aktivitäten der entsprechenden Muskelgruppe in der Körperperipherie, die nicht nur durch die mentale Stimulation erfolgt, sondern auch durch die propriozeptive Rückmeldung nach unbewusster Ansteuerung und entsprechender Reaktion der angesprochenen Muskulatur. Dies ist vor allem für die Nachhaltigkeit und Haltezeit der erreichten Muskelkräftigung von Bedeutung, nachdem damit jede natürliche Muskelkontraktion einer erhöhten zentralen Signalgebung unterliegt und somit mehr Muskelfasern angesprochen werden können. Damit gelingt es, einer im Alter üblichen zunehmenden Abkopplung motorischer Einheiten von der nervalen Versorgung entgegen zu wirken (Hughes VA, Frontera WR, Wood M et al. Longitudinal muscle strength changes in older adults: influence of muscle mass, physical activity, and health. J Geontol Biol Sci Med Sci. 2001; 56(5): B209-217).

Um demnach das volle Potential eines "passiven" rPMS-Trainings auszureizen, ist es auf Basis eines spezifischen "Visualisierungsprotokolls" notwendig, die rPMS-bedingten Kontraktionsserien der Beckenbodenmuskulatur jeweils mit dem Versuch einer Eigenkontraktion zu begleiten, wobei der Benutzer angehalten wird, sich eine starke Muskelbelastung des Beckenbodens vorzustellen. Eine Eigenkontraktion während eines rPMS-Trainings ist zwar ein frustranes Bemühen, da die elektromagnetisch induzierten Aktionspotentiale eine Eigenkontraktion verhindern. Eine korrekt durchgeführte Visualisierung führt aber zu einer erweiterten Prägung im Repräsentationszentrum in der Großhirnrinde, indem es dort zusätzlich "stille" und gehemmte synaptische Verbindungen aktiviert und verstärkt.

Ein High-Mental-Effort-Training (HMET), ist besonders dann besonders effektiv, wenn eine Person mit einer zu geringen MVC (Maximal Voluntary Contraction) von z.B. 30 % trainiert (Jiang CH et al. The level of effort, rather than muscle exercise intensity determines strength gain following a six-week training. Life Sci. 2017;178:30-34) trainiert, zumal hier einzelne, rPMS-erzwungene Muskelkontraktionen nicht limitierend für eine Stimulation mittels HMET sind. Davon profitieren vor allem Personen, die alters- oder auch sensibilitätsbedingt nicht bereit sind, ein rPMS-Training höherer Intensität durchzuführen. Werden nämlich dadurch nur Teile des Beckenbodens von der rPMS erfasst, unterbleibt die für ein Muskelwachstum und die zentrale Perzeption so wichtige globale Muskelkontraktion. Wird eine niederintensive rPMS stattdessen mit einem HMET kombiniert, gelingt es, das Intensitätsdefizit fast vollständig auszugleichen.

Die Visualisierungsprogramme beziehen sich nicht auf eine allgemeine Kontraktion des Beckenbodens, sondern unterscheiden sich entsprechend der jeweiligen Indikation. So unterliegt z.B. ein HMET für die Belastungsinkontinenz dem Tenor einer drohenden Inkontinenzsituation während körperlicher Belastung, während bei der Dranginkontinenz oder dem OAB eine Blockierung des Entleerungsreizes im gedanklichen Fokus steht. Schmerzsyndrome wiederum bauen auf der Auflösung von myofaszialen Verhärtungen (taut band) und muskulärer Knoten auf. Und die Visualisierung beim Core-Strength-Training orientiert sich am typischen Bewegungsablauf in der jeweiligen Sportart.

Um eine effektive Visualisierung durchzuführen, reicht es nicht aus, dies dem Benutzer per einmaliger Instruktion mitzugeben. In der Regel benötigt er eine kontinuierliche Vorgabe, die nur durch einen entsprechend geschulten Therapeuten oder durch eine animierte bildliche Taktgebung gelingt. Hierzu werden die Kontraktionsanweisungen des bildschirmpräsenten Animateurs mit dem Harninkontinenz- oder Core-Strength-Programm der rPMS elektronisch gekoppelt. So empfängt der Benutzer seine jeweiligen Kontraktionsanweisungen immer millisekundengenau zur Interventionszeit (in der Regel 8 sec) der rPMS, da sich eine Visualisierungsanweisung in der Pausenzeit der rPMS (4 sec) kontraproduktiv auswirken würde.

Die Benutzer können dabei per Touchscreen entscheiden, ob sie lieber eine männliche oder weibliche Stimme hören bzw. einem männlichen oder weiblichen Instruktor sehen wollen und ob die Stimmfarbe sehr tief, hell, warm oder auch dominant ertönt. Ebenso wird die Visualisierung in den gängigen Fremdsprachen bereitgehalten.

### Haltgriffe 8 für aktives Training

Ein rein mentales "High-Mental-Effort-Training" lässt sich verstärken, wenn der Benutzer seine gedanklichen Kontraktionsbemühungen mit dem Versuch einer Muskelanspannung kombiniert. Dies lässt sich idealerweise optimieren, indem der Benutzer mittels Ziehen bzw. Zugwiderstand durch zwei in die beidseitigen Armablagen integrierte Haltegriffe 8 sowohl die Bauch-, als auch die Beckenbodenmuskulatur zur Anspannung bringt und damit die betroffenen Muskeln hinsichtlich eines physiologischen Bewegungsablaufs koordiniert. Dieses aktive Widerstandstraining ist ausschließlich für ein Coretraining, die Sarkopenie, im Falle einer Belastungsinkontinenz sowie für spastisch gelähmte Benutzer (Querschnitts-lähmung, Schlaganfall, Multiple Sklerose) vorgesehen.

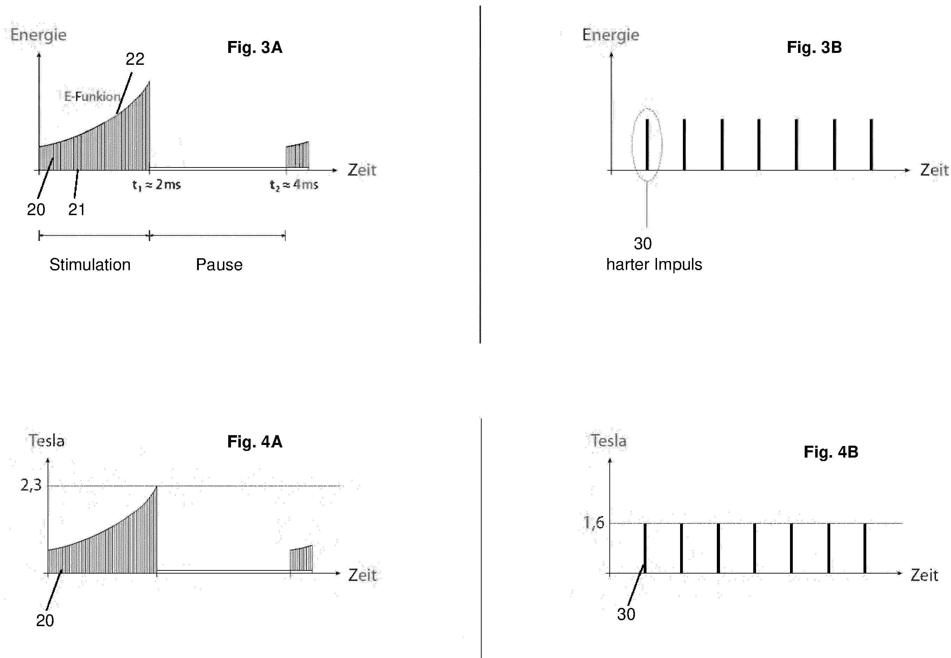

Die Figuren 3A und 3B zeigen schematisch einen Vergleich zwischen der Stärke (Energie) des Magnetfelds der erfindungsgemäßen Vorrichtung zur Magnetfeldstimulation und einer Magnetfeldstimulationsvorrichtung gemäß dem Stand der Technik.

In Figur 3A ist die Impulsform der erfindungsgemäßen Vorrichtung zur Magnetfeldstimulation dargestellt. Während der Stimulationszeit wird die Amplitude der einzelnen Magnetimpulse 20 stetig erhöht, vorzugsweise entsprechend einer Hüllkurve 22, die einer e-Funktion folgt. Die Hüllkurve 22 der Magnetimpulse beginnt vorzugsweise nicht bei null, sondern auf einem definierten Anfangswert. Die Amplitude steigt einer e-Funktion folgend bis auf einen Maximalwert und fällt dann abrupt auf null. Es folgt eine definierte Pausenzeit, die beispielsweise genauso lang sein kann wie die Stimulationszeit. Nach der Pausenzeit beginnt eine weitere Stimulationszeit.

Während der Stimulationszeit werden mehrere Impulsgruppen 21 abgeben, die jeweils aus mehreren Einzelimpulsen 20 bestehen. Die Einzelimpulse 20 der einzelnen Impulsgruppen 21 folgen der Hüllkurve 22.

Beim Stand der Technik gemäß Figur 3B erkennt man, dass mit kurzen Magnetimpulsen 30 gearbeitet wird, deren Maximalwert jeweils gleich groß ist. Die Frequenz der Impulse 30 ändert sich nicht. Es handelt sich um harte, direkte Impulse 30.

Figur 4A zeigt schematisch die magnetische Flussdichte des pulsierenden Magnetfelds über die Zeit bei der erfindungsgemäßen Vorrichtung zur Magnetfeldstimulation. Man erkennt, dass die magnetische Flussdichte der Impulse 20 entsprechend der Hüllkurve ansteigt und einen Maximalwert von beispielsweise 2,3 Tesla erreicht. Dadurch kann eine große Wirktiefe von bis zu 12 cm in das Körpergewebe des Benutzers erreicht werden.

Figur 4B zeigt schematisch die magnetische Flussdichte des pulsierenden Magnetfeldes über die Zeit bei einer Magnetfeldstimulationsvorrichtung gemäß dem Stand der Technik. Die Einzelimpulse 30 haben alle dieselbe Amplitude und erreichen beispielsweise eine maximale magnetische Flussdichte von 1,6 Tesla. Somit kann lediglich eine maximale Wirktiefe von 4,5 cm in das Körpergewebe des Benutzers erreicht werden.

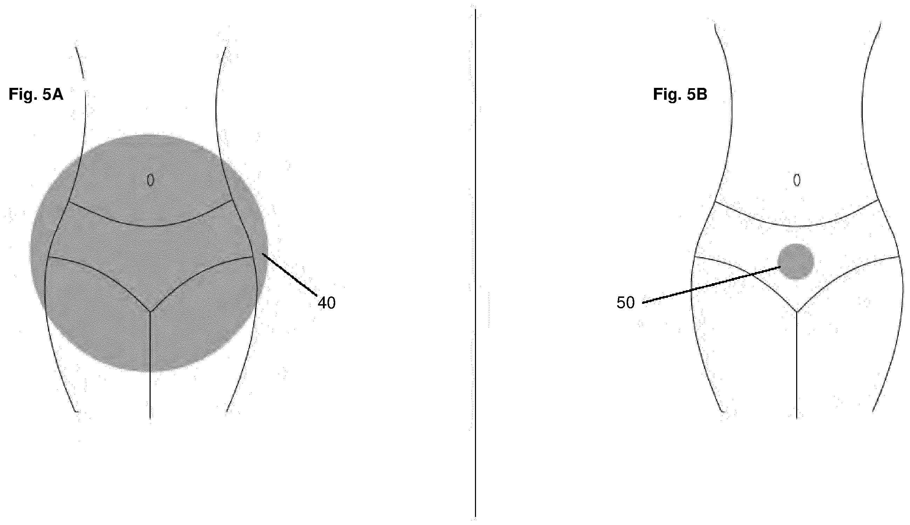

Figur 5A zeigt einen weiteren Vorteil der erfindungsgemäßen Vorrichtung zur Magnetfeldstimulation. Es ist schematisch die Wirkfläche 40 des Magnetfelds auf den Beckenbereich eines Benutzers dargestellt. Durch entsprechende Auslegung der Magnetspule 15 unterhalb der Sitzfläche 3 des Benutzers kann eine große Wirkfläche 40 des Magnetfeldes von beispielsweise 2400 mm² erreicht werden. Innerhalb diese Wirkfläche 40 herrscht eine relative gleichmäßige magnetische Flussdichte.

Figur 5B zeigt schematisch die typische Wirkfläche 50 des Magnetfelds bei einer Magnetfeldstimulationsvorrichtung gemäß dem Stand der Technik, die beispielsweise nur 12 mm² beträgt.

Die Wirkfläche kann erfindungsgemäß vergrößert werden, indem die Position der Magnetspule 15 in Bezug auf die Sitzfläche 3 verändert wird.

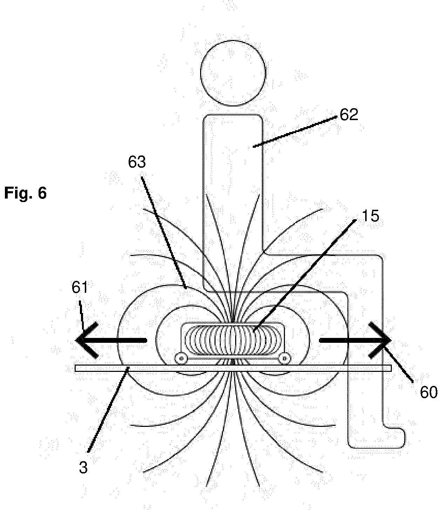

Figur 6 zeigt schematisch eine in Pfeilrichtungen verschiebbare Magnetspule 15 unterhalb der Sitzfläche 3 der Vorrichtung. Die Verschiebung der Magnetspule 15 kann vorzugsweise in Sitzrichtung des Benutzers 62 entsprechend den Pfeilen 60, 61 nach vorne und hinten erfolgen, oder aber ebenso nach links und rechts zur Seite oder entlang eines programmierten Bewegungspfades, z.B. entlang einer Kreisbahn.

Durch die Möglichkeit der Verschiebung der Magnetspule 15 in Bezug auf die Sitzfläche vergrößert sich die effektive Wirkfläche des Magnetfeldes 63 der Magnetspule 15, und es kann eine effektive Einwirkung des Magnetfeldes 63 auf den Beckenbodenbereich des Benutzers 62 erreicht werden, unabhängig vom individuellen Körperbau des Benutzers und seiner Sitzposition auf der Sitzfläche 3 der Vorrichtung.

Erfindungsgemäß erfolgt die Verschiebung der Magnetspule 15 in Bezug auf die Sitzfläche 3 automatisch zwischen beispielsweise eine vorderen und hinteren Position, d.h. während der Dauer der Magnetfeldstimulation bewegt sich die Magnetspule automatisch in Bezug auf die Sitzfläche 3 der Vorrichtung vor und zurück. Die Verschiebung der Magnetspule 15 kann periodisch erfolgen oder individuell gesteuert entsprechend einem vorgegebenen Zeitablauf.

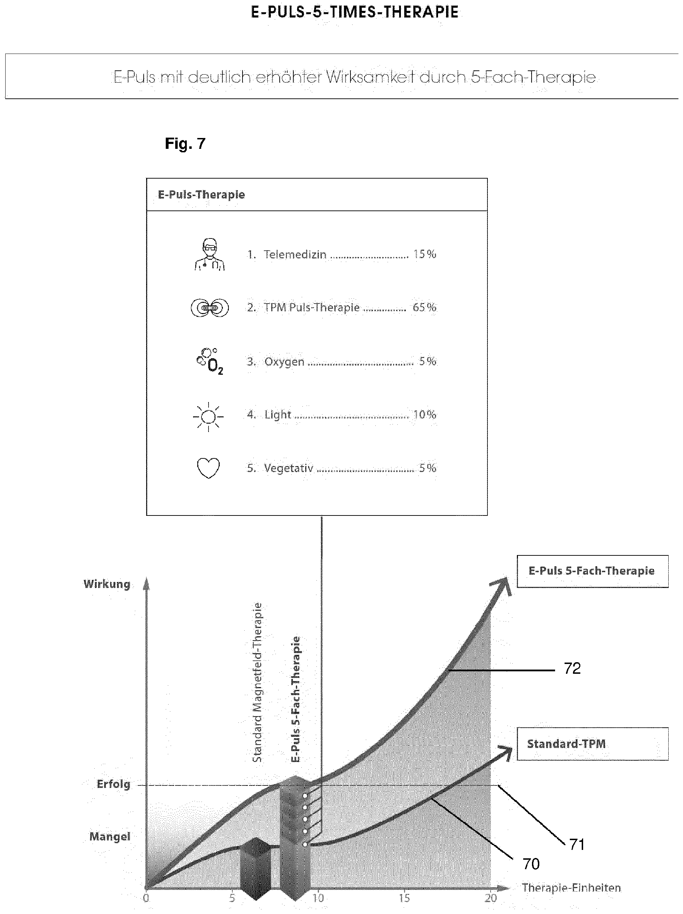

Figur 7 verdeutlicht schematisch die effektivere Wirkung der erfindungsgemäßen Vorrichtung zur Magnetfeldstimulation im Vergleich zum Stand der Technik. Beim Stand der Technik wird eine herkömmliche Magnetfeldstimulation angewendet, die - wie weiter oben beschrieben wurde - eine relativ kleine Wirkfläche und eine geringe Eindringtiefe aufweist. Dadurch stellt sich eine Gesamtwirkung 70 auf das Körpergewebe des Benutzers ein, die nur geringfügig über dem Schwellenwert 71 für eine spürbare Verbesserung des Wohlbefindens liegt.

Durch die erfindungsgemäße Vorrichtung zur Magnetfeldstimulation kann eine sehr viel bessere Wirkung 72 auf das Wohlbefinden erzeugt werden, wie sich aus Figur 7 ergibt. Hierfür wird eine kombinierte Magnetfeldstimulation eingesetzt, bei der nicht nur die QRS Magnetfeldstimulation verwendet wird, sondern bei der dem Benutzer zusätzlich Sauerstoff verabreicht werden kann. Ferner kann durch die vorhandenen Beleuchtungsmittel 12 gleichzeitig eine Lichteinwirkung auf den Benutzer erfolgen. Außerdem können die verwendeten Frequenzen für die Magnetfeldstimulation derart gesteuert werden, dass sich eine zusätzlich positive Wirkung auf das Herz-/Kreislaufsystem ergibt. Um dem Patienten einen höchstmöglichen therapeutischen Support zu bieten und um die Therapieeffekte noch weiter zu verstärken, wird die rPMS-Behandlung durch die exklusive Verfügbarkeit einer telemedizinischen Beratung begleitet.

Es erfolgt demnach eine 5-fach Einwirkung auf den Benutzer durch Magnetfeldstimulation, Sauerstoffverabreichung, Lichteinwirkung und Herz-Kreislauf Stimulation und Telemedizin. Durch die verbesserte Wirkung der 5-fach-Therapie kann die Behandlungszeit im Vergleich zur Standard-Magnetfeldstimulation verkürzt werden.

### Liste der Bezugszeichen

- 1: Grundgestell
- 2: Sitzgelegenheit
- 3: Sitzfläche
- 4: Rückenlehne
- 5: Fußteil
- 6: Armlehne
- 7: Bedienpult
- 8: Haltegriff
- 9: Lichthaube
- 10: Bildschirm
- 11: Lautsprecher
- 12: Beleuchtungsmittel
- 13: Mikrofon
- 14: Kamera
- 15: Magnetfeldapplikator

- 20: Einzelimpuls
- 21: Impulsgruppe
- 22: Hüllkurve
- 30: Impuls
- 40: Wirkfläche
- 50: Wirkfläche
- 60: Pfeilrichtung
- 61: Pfeilrichtung
- 62: Benutzer
- 63: Magnetfeld
- 70: Wirkung herkömmliche Magnetfeldstimulation
- 71: Schwellenwert
- 72: Wirkung der erfindungsgemäßen 5-fach-Magnetfeldstimulation

## Patentansprüche

1. Vorrichtung zur Magnetfeldstimulation von Körpergewebe eines Benutzers, welche umfasst:
eine einstellbare Sitzgelegenheit (2) für den Benutzer,
mindestens einen Magnetfeldapplikator (15) zur Erzeugung eines pulsierenden Magnetfeldes im Bereich der Sitzgelegenheit (2),
Einstellmittel (7) zur Einstellung eines oder mehrerer Parameter des pulsierenden Magnetfeldes umfassend Pulsdauer, Pulsfrequenz und Pulsamplitude, und zur Einstellung der Sitzgelegenheit (2), und Anzeigemittel (10) zur Anzeige von Informationen,
wobei die Einstellmittel (7) ausgebildet sind, um einen oder mehrere der Parameter des Magnetfeldes und/oder die Einstellung der Sitzgelegenheit (2) in Abhängigkeit von zuvor aufgenommenen individuellen Informationen über den Benutzer manuell und/oder automatisch einzustellen, **dadurch gekennzeichnet, dass** im Bereich der einstellbaren Sitzgelegenheit (2) Haltegriffe (8) für den Benutzer angeordnet sind die so ausgebildet sind, dass der Benutzer damit aktiv seine Muskulatur anspannen und den Behandlungserfolg steigern kann.

2. Vorrichtung nach Anspruch 1, **dadurch gekennzeichnet, dass** der Magnetfeldapplikator eine oder mehrere Magnetspulen (15) aufweist, die in einer Sitzfläche (3) und/oder einer Rückenlehne (4) und/oder einem Fußteil (5) der einstellbaren Sitzgelegenheit (2) angeordnet sind.

3. Vorrichtung nach einem der Ansprüche 1 oder 2, **dadurch gekennzeichnet, dass** eine Einstellung der einstellbaren Sitzgelegenheit (2) und/oder eine Einstellung der Parameter des pulsierenden Magnetfeldes automatisch anhand der über den Benutzer verfügbaren individuellen Informationen erfolgt.

4. Vorrichtung nach einem der Ansprüche 1 bis 3, **dadurch gekennzeichnet, dass** eine Vorrichtung (13, 14) zur automatischen Erkennung des Benutzers vorgesehen ist.

5. Vorrichtung nach einem der Ansprüche 1 bis 4, **dadurch gekennzeichnet, dass** die Einstellmittel (7) zur Eingabe und/oder zum Auslesen der individuellen Informationen über den Benutzer vorgesehen sind, insbesondere Angaben über körperliche Merkmale und/oder den Gesundheitszustand des Benutzers.

6. Vorrichtung nach Anspruch 5, **dadurch gekennzeichnet, dass** die individuellen Informationen über den aktuellen Benutzer mit Informationen über andere Benutzer verglichen werden, und die Parameter des Magnetfeldes für den aktuellen Benutzer unter Berücksichtigung von zuvor bei den anderen Benutzern eingestellten Parametern eingestellt werden.

7. Vorrichtung nach einem der Ansprüche 1 bis 6, **dadurch gekennzeichnet, dass** auf dem Anzeigemittel (10) eine Information über die Wirkungsweise von unterschiedlichen Einstellungen des pulsierenden Magnetfeldes auf das Körpergewebe darstellbar ist.

8. Vorrichtung nach einem der Ansprüche 1 bis 7, **dadurch gekennzeichnet, dass** eine Vorrichtung (12) zur Erzeugung von Beleuchtungs- und Lichteffekten vorgesehen ist.

9. Vorrichtung nach einem der Ansprüche 1 bis 8, **dadurch gekennzeichnet, dass** ein Infotainmentsystem (10, 11, 12, 13) zum Abspielen von Musik, Ton-, Licht- und Klangeffekten vorgesehen ist.

10. Vorrichtung nach einem der Ansprüche 1 bis 9, **dadurch gekennzeichnet, dass** ein Mikrophon (13) zur Aufnahme von Sprachinformationen zur Sprachsteuerung und/oder zur Erkennung des Benutzers vorgesehen ist.

11. Vorrichtung nach einem der Ansprüche 1 bis 10, **dadurch gekennzeichnet, dass** die Einstellmittel (7) eine berührungsempfindliche Anzeigevorrichtung aufweisen.

12. Vorrichtung nach einem der Ansprüche 1 bis 11, **dadurch gekennzeichnet, dass** die vom Benutzer aufgenommenen individuellen Informationen in der Vorrichtung selbst und/oder in einer Zentrale gespeichert und ausgewertet werden.

13. Vorrichtung nach einem der Ansprüche 1 bis 12, **dadurch gekennzeichnet, dass** die Magnetspule (15) in Bezug auf die Sitzfläche (3) beweglich angeordnet ist.

14. Vorrichtung nach Anspruch 13, **dadurch gekennzeichnet, dass** die Bewegung der Magnetspule (15) in Bezug auf die Sitzfläche (3) während der Magnetfeldstimulation durch einen Antrieb automatisch erfolgt, wobei die Bewegung der Magnetspule (15) automatisch zwischen zwei oder mehreren Endpositionen erfolgt oder einem vorgegeben Bewegungspfad folgend gesteuert ist.

15. Vorrichtung nach einem der Ansprüche 1 bis 14, **dadurch gekennzeichnet, dass** eine Atemmaske oder eine Nasenbrille zur Sauerstoffgabe an den Benutzer vorgesehen sind.

16. Vorrichtung nach einem der Ansprüche 1 bis 15, **dadurch gekennzeichnet, dass** ein Modul für eine telemedizinische Beratung vorgesehen ist.

## Claims

1. Device for magnetic field stimulation of body tissue of a user, which comprises:
an adjustable seat arrangement (2) for the user,
at least one magnetic field applicator (15) to generate a pulsating magnetic field in the region of the seat arrangement (2),
adjusting means (7) for adjusting one or more parameters of the pulsating magnetic field including pulse duration, pulse frequency and pulse amplitude, and for adjusting the seat arrangement (2), and
display means (10) to display information,
wherein the adjusting means (7) are designed in order to adjust one or more of the parameters of the magnetic field and/or adjustment of the seat arrangement (2) as a function of previously recorded individual information about the user manually and/or automatically,
**characterised in that** grips (8) for the user, which are designed so that the user may thus actively flex his muscular system and increase the success of treatment, are arranged in the region of the adjustable seat arrangement (2).

2. Device according to claim 1, **characterised in that** the magnetic field applicator has one or more magnetic coils (15) which are arranged in a seat surface (3) and/or a backrest (4) and/or a foot part (5) of the adjustable seat arrangement (2).

3. Device according to one of claims 1 or 2, **characterised in that** adjustment of the adjustable seat arrangement (2) and/or adjustment of the parameters of the pulsating magnetic field is effected automatically using the individual information available about the user.

4. Device according to one of claims 1 to 3, **characterised in that** a device (13, 14) is provided for automatic recognition of the user.

5. Device according to one of claims 1 to 4, **characterised in that** the adjusting means (7) are provided for inputting and/or for reading-out individual information about the user, in particular details about physical features and/or the health status of the user.

6. Device according to claim 5, **characterised in that** the individual information about the current user is compared with information about other users, and the parameters of the magnetic field for the current user are adjusted taking into account parameters adjusted beforehand for the other users.

7. Device according to one of claims 1 to 6, **characterised in that** information about the mode of action of different adjustments of the pulsing magnetic field on the body tissue can be shown on the display means (10).

8. Device according to one of claims 1 to 7, **characterised in that** a device (12) is provided to generate illumination and light effects.

9. Device according to one of claims 1 to 8, **characterised in that** an infotainment system (10, 11, 12, 13) is provided to play music, atmosphere, light and sound effects.

10. Device according to one of claims 1 to 9, **characterised in that** a microphone (13) is provided to record voice information for voice control and/or for recognising the user.

11. Device according to one of claims 1 to 10, **characterised in that** the adjusting means (7) have a touch-sensitive display device.

12. Device according to one of claims 1 to 11, **characterised in that** the individual information recorded by the user is stored and evaluated in the device itself and/or in a control centre.

13. Device according to one of claims 1 to 12, **characterised in that** the magnetic coil (15) is arranged to be movable relative the seat surface (3).

14. Device according to claim 13, **characterised in that** the movement of the magnetic coil (15) relative to the seat surface (3) is effected automatically by a drive during magnetic field stimulation, wherein the movement of the magnetic coil (15) is effected automatically between two or more end positions or is controlled following a predetermined movement path.

15. Device according to one of claims 1 to 14, **characterised in that** a breathing mask or a nasal cannula are provided to administer oxygen to the user.

16. Device according to one of claims 1 to 15, **characterised in that** a module is provided for telemedical advice.

## Revendications

1. Dispositif pour la stimulation par champ magnétique de tissu corporel d'un utilisateur, qui comprend :
un siège (2) réglable pour l'utilisateur,
au moins un applicateur de champ magnétique (15) pour production d'un champ magnétique pulsé dans la zone du siège (2),
des moyens de réglage (7) pour le réglage d'un ou plusieurs paramètres du champ magnétique pulsé, comprenant la durée d'impulsion, la fréquence d'impulsion et l'amplitude d'impulsion, et pour le réglage du siège (2), et
des moyens d'affichage (10) l'affichage d'informations,
dans lequel les moyens de réglage (7) sont réalisés pour régler manuellement et/ou automatiquement un ou plusieurs des paramètres du champ magnétique et/ou le réglage du siège (2) en fonction d'informations individuelles sur l'utilisateur enregistrées au préalable,
**caractérisé en ce que** des poignées de maintien (8) pour l'utilisateur sont disposées dans la zone du siège (2) réglable, lesquelles sont réalisées de telle sorte que l'utilisateur puisse ainsi contracter activement ses muscles et augmenter la réussite du traitement.

2. Dispositif selon la revendication 1, **caractérisé en ce que** l'applicateur de champ magnétique présente une ou plusieurs bobines magnétiques (15) qui sont disposées dans une surface d'assise (3) et/ou dans un dossier (4) et/ou dans une partie pour pieds (5) du siège (2) réglable.

3. Dispositif selon l'une des revendications 1 ou 2, **caractérisé en ce qu'**un réglage du siège (2) réglable et/ou un réglage des paramètres du champ magnétique pulsé sont effectués automatiquement à l'aide des informations individuelles disponibles sur l'utilisateur.

4. Dispositif selon l'une des revendications 1 à 3, **caractérisé en ce qu'**un dispositif (13, 14) est prévu pour la reconnaissance automatique de l'utilisateur.

5. Dispositif selon l'une des revendications 1 à 4, **caractérisé en ce que** les moyens de réglage (7) sont prévus pour l'entrée et/ou pour la lecture des informations individuelles sur l'utilisateur, en particulier des indications sur les caractéristiques physiques et/ou l'état de santé de l'utilisateur.

6. Dispositif selon la revendication 5, **caractérisé en ce que** les informations individuelles sur l'utilisateur du moment sont comparées à des informations sur d'autres utilisateurs, et les paramètres du champ magnétique pour l'utilisateur du moment sont réglés en tenant compte de paramètres réglés au préalable pour les autres utilisateurs.

7. Dispositif selon l'une des revendications 1 à 6, **caractérisé en ce qu'**une information sur le mode d'action de différents réglages du champ magnétique pulsé sur le tissu corporel peut être représentée sur le moyen d'affichage (10).

8. Dispositif selon l'une des revendications 1 à 7, **caractérisé en ce qu'**un dispositif (12) est prévu la production d'effets d'éclairage et de lumière.

9. Dispositif selon l'une des revendications 1 à 8, **caractérisé en ce qu'**un système d'info-divertissement (10, 11, 12, 13) est prévu pour jouer de la musique, des effets sonores, lumineux et de sonorité.

10. Dispositif selon l'une des revendications 1 à 9, **caractérisé en ce qu'**un microphone (13) est prévu pour l'enregistrement d'informations vocales, pour la commande vocale et/ou pour la reconnaissance de l'utilisateur.

11. Dispositif selon l'une des revendications 1 à 10, **caractérisé en ce que** les moyens de réglage (7) présentent un dispositif d'affichage tactile.

12. Dispositif selon l'une des revendications 1 à 11, **caractérisé en ce que** les informations individuelles enregistrées par l'utilisateur sont mémorisées et évaluées dans le dispositif lui-même et/ou dans une unité centrale.

13. Dispositif selon l'une des revendications 1 à 12, **caractérisé en ce que** la bobine magnétique (15) est disposée de manière mobile par rapport à la surface d'assise (3).

14. Dispositif selon la revendication 13, **caractérisé en ce que** le déplacement de la bobine magnétique (15) par rapport à la surface d'assise (3) est effectué automatiquement par l'entraînement au cours de la stimulation de champ magnétique, dans lequel le déplacement de la bobine magnétique (15) est effectué automatiquement entre deux ou plusieurs positions finales ou de manière à suivre un trajet de déplacement spécifié.

15. Dispositif selon l'une des revendications 1 à 14, **caractérisé en ce qu'**un masque respiratoire ou une lunette nasale pour l'administration d'oxygène sont prévus sur l'utilisateur.

16. Dispositif selon l'une des revendications 1 à 15, **caractérisé en ce qu'**un module est prévu pour une téléconsultation médicale.
